(19) Europäisches Patentamt
European Patent Office
Office européen des brevets

(11) EP 1 762 559 A1

(12) EUROPEAN PATENT APPLICATION
published in accordance with Art. 158(3) EPC

(43) Date of publication:
14.03.2007 Bulletin 2007/11

(51) Int Cl.:
C07C 68/06 (2006.01)      B01D 3/14 (2006.01)
C07C 69/96 (2006.01)      C07B 61/00 (2006.01)

(21) Application number: 05751392.1

(22) Date of filing: 20.06.2005

(86) International application number:
PCT/JP2005/011280

(87) International publication number:
WO 2006/001256 (05.01.2006 Gazette 2006/01)

(84) Designated Contracting States:
AT BE BG CH CY CZ DE DK EE ES FI FR GB GR HU IE IS IT LI LT LU MC NL PL PT RO SE SI SK TR

(30) Priority: 25.06.2004 JP 2004188464

(71) Applicant: Asahi Kasei Chemicals Corporation
Tokyo 100-8440 (JP)

(72) Inventors:
• FUKUOKA, Shinsuke
  Chiyoda-ku, Tokyo 100- 8440 (JP)
• HACHIYA, Hiroshi
  Chiyoda-ku, Tokyo 100- 8440 (JP)
• MATSUZAKI, Kazuhiko
  Chiyoda-ku, Tokyo 100- 8440 (JP)

(74) Representative: Strehl Schübel-Hopf & Partner
Maximilianstrasse 54
80538 München (DE)

(54) PROCESS FOR PRODUCING AROMATIC CARBONATE ON COMMERCIAL SCALE

(57)     It is an object of the present invention to provide a specific process that enables an aromatic carbonate to be produced with high selectivity and high productivity stably for a prolonged period of time on an industrial scale of no less than 1 ton per hour using a continuous multi-stage distillation column from a dialkyl carbonate and an aromatic monohydroxy compound containing specified amounts of an alcohol and an aromatic carbonate. Although there have been many proposals regarding the process of producing the aromatic carbonate using a re-active distillation method, these have all been on a small scale and a short operating time laboratory level. There have also been no disclosures whatsoever of a starting material containing necessary amounts of the alcohol and the aromatic carbonate for use in an industrial production, which have been considered to be disadvantageous in terms of the equilibrium, let alone the process and apparatus for the industrial production. According to the present invention, there is provided a specified continuous multi-stage distillation column, and there is also provided a specific process that enables the aromatic carbonate to be produced with high selectivity and high productivity stably for a prolonged period of time on an industrial scale of not less than 1 ton per hour from the dialkyl carbonate and the aromatic monohydroxy compound containing the alcohol and the aromatic carbonate.

FIG.1

**Description**

Technical Field

The present invention relates to an industrial process for the production of an aromatic carbonate. More particularly, the present invention relates to an industrial process for the production of a large amount of the aromatic carbonate useful as a raw material of polycarbonate for transesterification method by subjecting a dialkyl carbonate and an aromatic monohydroxy compound to transesterification in a continuous multi-stage distillation column in which a catalyst is present.

Background Art

An aromatic carbonate is important as a raw material for the production of an aromatic polycarbonate which is the most widely used as engineering plastic, without using toxic phosgene. As a process for producing an aromatic carbonate, a process reacting an aromatic monohydroxy compound with phosgene has been known from long ago, and has also been the subject of a variety of studies in recent years. However, this process has the problem of using phosgene, and in addition chlorinated impurities which are difficult to separate out are present in the aromatic carbonate produced using this process, and hence this aromatic carbonate cannot be used as the raw material for the production of aromatic polycarbonate. Because, such chlorinated impurities markedly inhibit the polymerization reaction in the transesterification method which is carried out in the presence of an extremely small amount of a basic catalyst; for example, even if such chlorinated impurities are present in an amount of only 1 ppm, the polymerization hardly proceed at all. To make the aromatic carbonate capable of using as a raw material of polycarbonate for transesterification method, troublesome multi-stage separation/purification processes such as enough washing with a dilute aqueous alkaline solution and hot water, oil/water separation, distillation and so on are required. Furthermore, the yield of the aromatic carbonate decreases due to hydrolysis loss and distillation loss during this separation/purification processes. Therefore, there are many problems in carrying out this method economically on an industrial scale.

On the other hand, a process for producing an aromatic carbonate through transesterification reactions between a dialkyl carbonate and an aromatic monohydroxy compound is also known. However, such transesterification reactions are all equilibrium reactions. Since the equilibriums are biased extremely toward the original system and the reaction rates are slow, there have been many difficulties in producing the aromatic carbonates industrially in large amounts using this method. Several proposals have been made to improve the above difficulties, but most of these have related to development of a catalyst to increase the reaction rate. Many metal compounds have been proposed as catalysts for this type of transesterification. For example, a Lewis acid such as a transition metal halide, and Lewis acid-forming compounds (See Patent Documents1: Japanese Patent Application Laid-Open No. 51-105032, Japanese Patent Application Laid-Open No. 56-123948, Japanese Patent Application Laid-Open No. 56-123949 (corresponding to West German Patent Application No. 2528412, British Patent No. 1499530, and U.S. Patent No. 4182726), Japanese Patent Application Laid-Open No. 51-75044 (corresponding to West German Patent Application No. 2552907, and U.S. Patent No. 4045464)), a tin compound such as an organotin alkoxide and an organotin oxide (See Patent Documents 2: Japanese Patent Application Laid-Open No. 54-48733 (corresponding to West German Patent Application No. 2736062), Japanese Patent Application Laid-Open No. 54-63023, Japanese Patent Application Laid-Open No. 60-169444 (corresponding to U.S. Patent No. 4554110), Japanese Patent Application Laid-Open No. 60-169445 (corresponding to U.S. Patent No. 4552704), Japanese Patent Application Laid-Open No. 62-277345, Japanese Patent Application Laid-Open No. 1-265063, Japanese Patent Application Laid-Open No. 60-169444 (corresponding to U.S. Patent No. 4554110), Japanese Patent Application Laid-Open No. 60-169445 (corresponding to U.S. Patent No. 4552704), Japanese Patent Application Laid-Open No. 62-277345, Japanese Patent Application Laid-Open No. 1-265063), salts and alkoxides of alkali metals and alkaline earth metals (See Patent Document 3: Japanese Patent Application Laid-Open No. 57-176932), lead compounds (See Patent Documents 4: Japanese Patent Application Laid-Open No. 57-176932, Japanese Patent Application Laid-Open No. 1-93560), complexes of a metal, such as copper, iron and zirconium (See Patent Document 5: Japanese Patent Application Laid-Open No. 57-183745), titanic acid esters (See Patent Documents 6: Japanese Patent Application Laid-Open No. 58-185536 (corresponding to U.S. Patent No. 4410464), Japanese Patent Application Laid-Open No. 1-265062), a mixture of a Lewis acid and protonic acid (See Patent document 7: Japanese Patent Application Laid-Open No. 60-173016 (corresponding to U.S. Patent No. 4609501)), a compound of Sc, Mo, Mn, Bi, Te or the like (See Patent Document 8: Japanese Patent Application Laid-Open No. 1-265064,), ferric acetate (See Patent Document 9: Japanese Patent Application Laid-Open No. 61-172852), and so on have been proposed. However, the problem of the disadvantageous equilibrium cannot be solved merely by developing the catalyst, and hence there are very many issues to be solved including the reaction system in order to provide a process for the industrial production aiming for mass production.

Attempts have also been made to devise a reaction system so as to bias the equilibrium toward the product system as much as possible, and thus improve the yield of the aromatic carbonates. For example, for the reaction between dimethyl carbonate and phenol, there have been proposed a method in which by-produced methanol is distilled off by azeotropy together with an azeotrope-forming agent (See Patent Document 10: Japanese Patent Application Laid-Open No. 54-48732 (corresponding to West German Patent Application No. 736063, and U.S. Patent No. 4252737)), and a method in which the methanol produced as the by-product is removed by being absorbed onto a molecular sieve (See Patent Document 11: Japanese Patent Application Laid-Open No. 58-185536 (corresponding to U.S. Patent No. 410464)). Moreover, a method has also been proposed in which, using an apparatus in which a distillation column is provided on the top of a reactor, an alcohol produced as the by-product in the reaction is separated off from the reaction mixture, and at the same time an unreacted starting material that evaporates is separated off by distillation (See Patent Documents 12: examples in Japanese Patent Application Laid-Open No. 56-123948 (corresponding to U.S. Patent No. 4182726), examples in Japanese Patent Application Laid-Open No. 56-25138, examples in Japanese Patent Application Laid-Open No. 60-169444 (corresponding to U.S. Patent No. 4554110), examples in Japanese Patent Application Laid-Open No. 60-169445 (corresponding to U.S. Patent No. 4552704), examples in Japanese Patent Application Laid-Open No. 60-173016 (corresponding to U.S. Patent No. 4609501), examples in Japanese Patent Application Laid-Open No. 61-172852, examples in Japanese Patent Application Laid-Open No. 61-291545, examples in Japanese Patent Application Laid-Open No. 62-277345)).

However, these reaction systems are basically batch system or switchover system. Because, there is the limitation in the improvement of the reaction rate through the catalyst development for such a transesterification reaction, and the reaction rate is still slow, it has been thought that the batch system is preferable to a continuous system. Of these, a continuous stirring tank reactor (CSTR) system in which a distillation column is provided on the top of the reactor has been proposed as the continuous system, but there are problems such as the reaction rate being slow, and a gas-liquid interface in the reactor being small, based on the volume of the liquid. Hence it is not possible to make the reaction ratio high. Accordingly, it is difficult to attain the object of producing the aromatic carbonate continuously in large amounts stably for a prolonged period of time by means of these above-mentioned methods, and many issues remain to be resolved before economical industrial implementation is possible.

The present inventors have developed reactive distillation methods in which such a transesterification reaction is carried out in a continuous multi-stage distillation column simultaneously with separation by distillation, and have been the first in the world to disclose that such a reactive distillation system is useful for such a transesterification reaction, for example, a reactive distillation method in which a dialkyl carbonate and an aromatic hydroxy compound are continuously fed into the multi-stage distillation column, the reaction is carried out continuously inside the column in which a catalyst is present, while continuously withdrawing a low boiling point component containing an alcohol produced as a by-product by distillation and continuously withdrawing a component containing a produced alkyl aryl carbonate from a lower portion of the column (See Patent Document 13: Japanese Patent Application Laid-Open No. 3-291257), a reactive distillation method in which an alkyl aryl carbonate is continuously fed into the multi-stage distillation column, the reaction is carried out continuously inside the column in which a catalyst is present, while continuously withdrawing by distillation a low boiling point component containing a dialkyl carbonate produced as a by-product and continuously withdrawing a component containing a produced diaryl carbonate from a lower portion of the column (See Patent Document 14: Japanese Patent Application Laid-Open No. 4-9358), a reactive distillation method in which these reactions are carried out using two continuous multi-stage distillation columns, and hence a diaryl carbonate is produced continuously while efficiently recycling a dialkyl carbonate produced as a by-product (See Patent Document 15: Japanese Patent Application Laid-Open No. 4-211038), and a reactive distillation method in which a dialkyl carbonate and an aromatic hydroxy compound or the like are continuously fed into the multi-stage distillation column, and a liquid that flows down through the column is withdrawn from a side outlet provided at an intermediate stage and/or a lowermost stage of the distillation column, and is introduced into a reactor provided outside the distillation column so as to bring about reaction, and is then introduced back through a circulating inlet provided at a stage above the stage where the outlet is provided, whereby reaction is carried out in both the reactor and the distillation column (See Patent Documents 16: Japanese Patent Application Laid-Open No. 4-224547, Japanese Patent Application Laid-Open No. 4-230242, Japanese Patent Application Laid-open No. 4-235951).

These reactive distillation methods proposed by the present inventors are the first to enable aromatic carbonates to be produced continuously and efficiently, and many similar reactive distillation systems based on the above disclosures have been proposed thereafter (See Patent Documents 17 to 32: Patent Document 17: International Publication No. 00/18720 (corresponding to U.S. Patent No. 5362901), Patent Document 18: Italian Patent No. 01255746, Patent Document 19: Japanese Patent Application Laid-Open No. 6-9506 (corresponding to European Patent No. 0560159, and U.S. Patent No. 5282965), Patent Document 20: Japanese Patent Application Laid-Open No. 6-41022 (corresponding to European Patent No. 0572870, and U.S. Patent No. 5362901), Patent Documents 21: Japanese Patent Application Laid-Open No. 6-157424 (corresponding to European Patent No. 0582931, and U.S. Patent No. 5334742), Japanese Patent Application Laid-Open No. 6-184058 (corresponding to European Patent No. 0582930, and U.S. Patent No. 5344954)), Patent Document 22: Japanese Patent Application Laid-Open No. 7-304713, Patent Document 23: Japanese Patent Application Laid-Open No. 9-40616, Patent Document 24: Japanese Patent Application Laid-Open No. 9-59225, Patent Document 25: Japanese Patent Application Laid-Open No. 9-110805, Patent Document 26: Japanese Patent Application Laid-Open No. 9-165357, Patent Document 27: Japanese Patent Application Laid-Open No. 9-173819, Patent Documents 28: Japanese Patent Application Laid-Open No. 9-176094, Japanese Patent Application Laid-Open No. 2000-191596, Japanese Patent Application Laid-Open No. 2000-191597, Patent Document 29: Japanese Patent Application Laid-Open No. 9-194436 (corresponding to European Patent No. 0785184, and U.S. Patent No. 5705673), Patent Document 30: International Publication No. 00/18720 (corresponding to U.S. Patent No. 6093842), Patent Documents 31: Japanese Patent Application Laid-Open No. 2001-64234, Japanese Patent Application Laid-Open No. 2001-64235, Patent Documents 32: International Publication No. 02/40439 (corresponding to U.S. Patent No. 6596894, U.S. Patent No. 6596895, U.S. Patent No. 6600061)).

Among reactive distillation systems, the present applicants have further proposed, as a method that enables highly pure aromatic carbonates to be produced stably for a prolonged period of time without a large amount of a catalyst being required, a method in which a high boiling point material containing a catalyst component is reacted with an active substance and then separated off, and the catalyst component is recycled (See Patent Documents 31: Japanese Patent Application Laid-Open No. 2001-64234, Japanese Patent Application Laid-Open No. 2001-64235), and a method carried out while keeping a weight ratio of a polyhydric aromatic hydroxy compound in the reaction system to a catalyst metal at not more than 2.0 (See Patent Documents 32: International Publication No. 02/40439 (corresponding to U.S. Patent No. 6596894, U.S. Patent No. 6596895, and U.S. Patent No. 6600061)). Furthermore, the present inventors have proposed a method in which 70 to 99 % by weight of phenol produced as a by-product in a polymerization process is used as a starting material, and diphenyl carbonate can be produced by means of the reactive distillation method. This diphenyl carbonate can be used as the raw material for polymerization of aromatic polycarbonates. (See Patent Document 33: International Publication No. 97/11049 (corresponding to European Patent No. 0855384, and U.S. Patent No. 5872275)).

However, in all of these prior art documents in which the production of the aromatic carbonates using the reactive distillation method is proposed, there is no disclosure whatsoever of a specific process or apparatus enabling mass production on an industrial scale (e.g. 1 ton per hour), nor is there any description suggesting such a process or apparatus. For example, the descriptions regarding a height (H: cm), a diameter (D: cm), and a number of stages (n) of the reactive distillation column and a feeding rate of the raw materials (Q: kg/hr) disclosed for producing mainly methyl phenyl carbonate (MPC) from dimethyl carbonate and phenol are summarized in the following Table 1.

Table 1

| H:cm | D:cm | NUMBER OF STAGE: n | Q:kg/hr | PATENT DOCUMENT |
|---|---|---|---|---|
| 60 | 5 | 10 | 0.5 | 19 |
| 200 | 5 | 20 | 1 | 20 |
| 350 | 2.8 | - | 0.2 | 21 |
| 500 | 5 | 50 | 0.6 | 23 |
| 100 | 4 | - | 1.4 | 24 |
| 300 | 5 | 40 | 1.5 | 26 |
| 300 | 5 | 40 | 1.5 | 28 |
| 400 | 12 | 40 | 53 | 29 |
| 340 | 5 | 40 | 2.3 | 32 |
| 1200 | 20 | 40 | 86 | 33 |
| 1200 | 20 | 40 | 86 | 34 |
| 600 | - | 20 | 66 | 35 |

See Patent Document 35: Japanese Patent Application Laid-Open No. 9-255772 (corresponding to European Patent No. 0892001, and U.S. Patent No. 5747609)

In other words, the biggest continuous multi-stage distillation columns used when carrying out this reaction using the reactive distillation system are those disclosed by the present applicants in Patent Documents 33 and 34. As can be shown from Table 1, the maximum values of the various conditions for the continuous multi-stage distillation columns disclosed for the above reaction are H = 1200 cm, D = 20 cm, n = 50 (Patent document 23), and Q = 86 kg/hr, and the total amount of aromatic carbonates produced (methyl phenyl carbonate and diphenyl carbonate combined) was only approximately 10 kg/hr, which was not an amount produced on an industrial scale.

Disclosure of Invention

It is an object of the present invention to provide a specific process that enables an aromatic carbonate to be produced with high selectivity and high productivity stably for a prolonged period of time on an industrial scale of no less than 1 ton per hour using a continuous multi-stage distillation column from a dialkyl carbonate and an aromatic monohydroxy compound containing specified amounts of an alcohol and the aromatic carbonate.

Since the present inventors disclose a process of producing aromatic carbonates using the continuous multi-stage distillation column, various proposals regarding the process for the production of the aromatic carbonates by means of the reactive distillation method have been made. However, these have all been on a small scale and a short operating time laboratory level, and there have been no disclosures whatsoever on a specific process or apparatus enabling mass production on an industrial scale. Moreover, there have been no disclosures whatsoever of a starting material containing necessary amounts of an alcohol and an aromatic carbonate for use in an industrial production, which have been considered to be disadvantageous in terms of the equilibrium, let alone the process and apparatus for the industrial production. In view of these circumstances, the present inventors carried out studies aimed at discovering a specific process enabling the aromatic carbonate to be produced with high selectivity and high productivity stably for a prolonged period of time on an industrial scale of not less than 1 ton per hour. As a result, the present inventors have reached to the present invention.

That is, in the first aspect of the present invention, there is provided:

1. A process for the production of an aromatic carbonate from a dialkyl carbonate and an aromatic monohydroxy compound as a starting material, which comprises the steps of:

(i) continuously feeding the starting material into a continuous multi-stage distillation column in which a catalyst is present;
(ii) carrying out reaction in the column to produce an alcohol and at least one aromatic carbonate; and
(iii) continuously withdrawing a low boiling point reaction mixture containing the produced alcohol from an upper portion of the column in a gaseous form, and continuously withdrawing a high boiling point reaction mixture containing the at least one aromatic carbonate from a lower portion of the column in a liquid form, wherein

(a) the starting material

(1) has a molar ratio of the dialkyl carbonate to the aromatic monohydroxy compound in a range of from 0.4 to 4, and
(2) comprises 0.01 to 1 % by weight of the alcohol, and 0.01 to 5 % by weight of the aromatic carbonate, based on the total weight of the starting material;

(b) the continuous multi-stage distillation column comprises a structure having a pair of end plates above and below a cylindrical trunk portion having a length L (cm) and an inside diameter D (cm) and having an internal with a number of stages n thereinside, and comprises a gas outlet having an inside diameter $d_1$ (cm) at the top of the column or in an upper portion of the column near thereto, a liquid outlet having an inside diameter $d_2$ (cm) at the bottom of the column or in a lower portion of the column near thereto, at least one inlet provided in the upper portion and/or a central portion of the column below the gas outlet, and at least one inlet provided in the lower portion of the column above the liquid outlet, wherein

(1) the length L (cm) satisfies following formula (1),

$$1500 \leqq L \leqq 8000 \qquad\qquad (1),$$

(2) the inside diameter D (cm) of the column satisfies the following formula (2),

$$100 \leqq D \leqq 2000 \qquad\qquad (2),$$

(3) a ratio of the length L (cm) to the inside diameter D (cm) of the column satisfies the following formula (3),

$$2 \leqq L / D \leqq 40 \qquad (3),$$

(4) the number of stages n satisfies the following formula (4),

$$20 \leqq n \leqq 120 \qquad (4),$$

(5) a ratio of the inside diameter D (cm) of the column to the inside diameter $d_1$ (cm) of the gas outlet satisfies the following formula (5),

$$5 \leqq D / d_1 \leqq 30 \qquad (5),$$

and
(6) a ratio of the inside diameter D (cm) of the column to the inside diameter $d_2$ (cm) of the liquid outlet satisfies the following formula (6),

$$3 \leqq D / d_2 \leqq 20 \qquad (6).$$

2. The method according to item 1, wherein distillation is carried out simultaneously in the step (ii).
3. The method according to item 1 or 2, wherein the at least one aromatic carbonate is continuously produced and an amount of the aromatic carbonate produced is not less than 1 ton per hour.
In another aspect of the process according to the present invention, there is provided:
4. A process for the industrial production of an aromatic carbonate in which at least one aromatic carbonate is produced continuously by continuously feeding a dialkyl carbonate and an aromatic monohydroxy compound as a starting material into a continuous multi-stage distillation column in which a catalyst is present, carrying out reaction and distillation simultaneously in the column, continuously withdrawing a low boiling point reaction mixture containing a produced alcohol from-an upper portion of the column in a gaseous form, and continuously withdrawing a high boiling point reaction mixture containing the at least one aromatic carbonate from a lower portion of the column in a liquid form, the improvement in which

(a) the starting material fed continuously into the continuous multi-stage distillation column

(1) has a molar ratio of the dialkyl carbonate to the aromatic monohydroxy compound in a range of from 0.4 to 4, and
(2) comprises 0.01 to 1 % by weight of the alcohol, and 0.01 to 5 % by weight of the aromatic carbonate, based on the total weight of the starting material;

(b) the continuous multi-stage distillation column comprises a structure having a pair of end plates above and below a cylindrical trunk portion having a length L (cm) and an inside diameter D (cm) and having an internal with a number of stages n thereinside, and comprises a gas outlet having an inside diameter $d_1$ (cm) at the top of the column or in an upper portion of the column near thereto, a liquid outlet having an inside diameter $d_2$ (cm) at the bottom of the column or in a lower portion of the column near thereto, at least one inlet provided in the upper portion and/or a central portion of the column below the gas outlet, and at least one inlet provided in the lower portion of the column above the liquid outlet, wherein

(1) the length L (cm) satisfies following formula (1),

$$1500 \leqq L \leqq 8000 \qquad (1),$$

(2) the inside diameter D (cm) of the column satisfies the following formula (2),

$$100 \leqq D \leqq 2000 \qquad (2),$$

(3) a ratio of the length L (cm) to the inside diameter D (cm) of the column satisfies the following formula (3),

$$2 \leqq L/D \leqq 40 \qquad (3),$$

(4) the number of stages n satisfies the following formula (4),

$$20 \leqq n \leqq 120 \qquad (4),$$

(5) a ratio of the inside diameter D (cm) of the column to the inside diameter $d_1$ (cm) of the gas outlet satisfies the following formula (5),

$$5 \leqq D/d_1 \leqq 30 \qquad (5),$$

and
(6) a ratio of the inside diameter D (cm) of the column to the inside diameter $d_2$ (cm) of the liquid outlet satisfies the following formula (6);

$$3 \leqq D/d_2 \leqq 20 \qquad (6).$$

5. The method according to item 4, wherein an amount of the aromatic carbonate produced is not less than 1 ton per hour.
6. The method according to any one of items 1 to 5, wherein the starting material further comprises 0.5 to 15 % by weight of an alkyl aryl ether, based on the total weight of the starting material.
7. The method according to any one of items 1 to 6, wherein $d_1$ and $d_2$ satisfy the following formula (7):

$$1 \leqq d_2/d_1 \leqq 5 \qquad (7).$$

8. The method according to any one of items 1 to 7, wherein L, D, L/D, n, $D/d_1$. and $D/d_2$ for the continuous multi-stage distillation column satisfy the following formulae; $2000 \leqq L \leqq 6000$, $150 \leqq D \leqq 1000$, $3 \leqq L/D \leqq 30$, $30 \leqq n \leqq 100$, $8 \leqq D/d_1 \leqq 25$, and $5 \leqq D/d_2 \leqq 18$, respectively.
9. The method according to any one of items 1 to 8, wherein L, D, L/D, n, $D/d_1$, and $D/d_2$ for the continuous multi-stage distillation column satisfy the following formulae; $2500 \leqq L \leqq 5000$, $200 \leqq D \leqq 800$, $5 \leqq L/D \leqq 15$, $40 \leqq n \leqq 90$, $10 \leqq D/d_1 \leqq 25$, and $7 \leqq D/d_2 \leqq 15$, respectively.
10. The method according to any one of items 1 to 9, wherein the continuous multi-stage distillation column is a distillation column having a tray and/or a packing as the internal.
11. The method according to items 10, wherein the continuous multi-stage distillation column is a plate-type distillation column having a tray as the internal.
12. The method according to item 10 or 11, wherein the tray is a sieve tray having a sieve portion and a down corner portion.

13. The method according to item 12, wherein the sieve tray has 100 to 1000 holes/m² in the sieve portion.

14. The method according to item 12 or 13, wherein the cross-sectional area per hole of the sieve tray is in a range of from 0.5 to 5 cm².

In the second aspect of the present invention, there is provided:

15. An aromatic carbonate comprising a halogen content of not more than 0.1 ppm, produced by the process according to any one of items 1 to 14.

In the third aspect of the present invention, there is provided:

16. A continuous multi-stage distillation column for carrying out reaction and distillation, comprising:

a cylindrical trunk portion having a length L (cm) and an inside diameter D (cm);
a pair of end plates provided above and below the trunk portion;
an internal with a number of stages n inside the trunk portion;
a gas outlet which has an inside diameter $d_1$ (cm), and which being provided on the end plate at the top of the column or in an upper portion of the column near thereto;
a liquid outlet which has an inside diameter $d_2$ (cm), and which being provided on the end plate at the bottom of the column or in a lower portion of the column near thereto;
at least one inlet provided in the upper portion and/or a central portion of the column below the gas outlet; and
at least one inlet provided in the lower portion of the column above the liquid outlet,
wherein

(1) the length L (cm) satisfies following formula (1),

$$1500 \leqq L \leqq 8000 \qquad (1),$$

(2) the inside diameter D (cm) of the column satisfies the following formula (2),

$$100 \leqq D \leqq 2000 \qquad (2),$$

(3) a ratio of the length L (cm) to the inside diameter D (cm) of the column satisfies the following formula (3),

$$2 \leqq L / D \leqq 40 \qquad (3),$$

(4) the number of stages n satisfies the following formula (4),

$$20 \leqq n \leqq 120 \qquad (4),$$

(5) a ratio of the inside diameter D (cm) of the column to the inside diameter $d_1$ (cm) of the gas outlet satisfies the following formula (5),

$$5 \leqq D / d_1 \leqq 30 \qquad (5),$$

and
(6) a ratio of the inside diameter D (cm) of the column to the inside diameter $d_2$ (cm) of the liquid outlet satisfies the following formula (6),

$$3 \leqq D / d_2 \leqq 20 \qquad (6).$$

17. The column according to item 16, wherein $d_1$ and $d_2$ satisfy the following formula (7):

$$1 \leqq d_2 / d_1 \leqq 5 \qquad (7).$$

18. The column according to item 16 or 17, wherein L, D, L /D, n, D /$d_1$, and D / $d_2$ for the continuous multi-stage distillation column satisfy the following formulae; $2000 \leqq L \leqq 6000$, $150 \leqq D \leqq 1000$, $3 \leqq L/D \leqq 30$, $30 \leqq n \leqq 100$, $8 \leqq D/d_1 \leqq 25$, and $5 \leqq D/d_2 \leqq 18$, respectively.

19. The column according to any one of items 16 to 18, wherein L, D, L /D, n, D / $d_1$, and D / $d_2$ for the continuous multi-stage distillation column satisfy the following formulae; $2500 \leqq L \leqq 5000$, $200 \leqq D \leqq 800$, $5 \leqq L/D \leqq 15$, $40 \leqq n \leqq 90$, $10 \leqq D/d_1 \leqq 25$, and $7 \leqq D/d_2 \leqq 15$, respectively.

20. The column according to any one of items 16 to 19, wherein the continuous multi-stage distillation column is a distillation column having a tray and/or a packing as the internal.

21. The column according to item 20, wherein the continuous multi-stage distillation column is a plate-type distillation column having a tray as the internal.

22. The column according to item 20 or 21, wherein the tray is a sieve tray having a sieve portion and a down corner portion.

23. The column according to item 22, wherein the sieve tray has 100 to 1000 holes/$m^2$ in the sieve portion.

24. The column according to item 22 or 23, wherein the cross-sectional area per hole of the sieve tray is in a range of from 0.5 to 5 $cm^2$.

Advantageous Effects of the Invention

Regarding the production of aromatic carbonates, even if there is used the dialkyl carbonate and the aromatic monohydroxy compound containing an alcohol and the aromatic carbonate as a starting material, which has been considered to be disadvantageous for the production of the aromatic carbonates, it has been discovered that by implementing the present invention which is characterized in that the molar ratio of a dialkyl carbonate to an aromatic monohydroxy compound which are used as a stating material is in a specified range, the contents of the alcohol and the aromatic carbonate are in a specified range and a specified continuous multi-stage distillation column is used, the aromatic carbonate can be produced on an industrial scale of not less than 1 ton per hour, preferably not less than 2 tons per hour, more preferably not less than 3 tons per hour, with high selectivity of not less than 95%, preferably not less than 97%, more preferably not less than 99%, stably for a prolonged period of time of not less than 2000 hours, preferably not less than 3000 hours, more preferably not less than 5000 hours.

Brief Description of Drawing

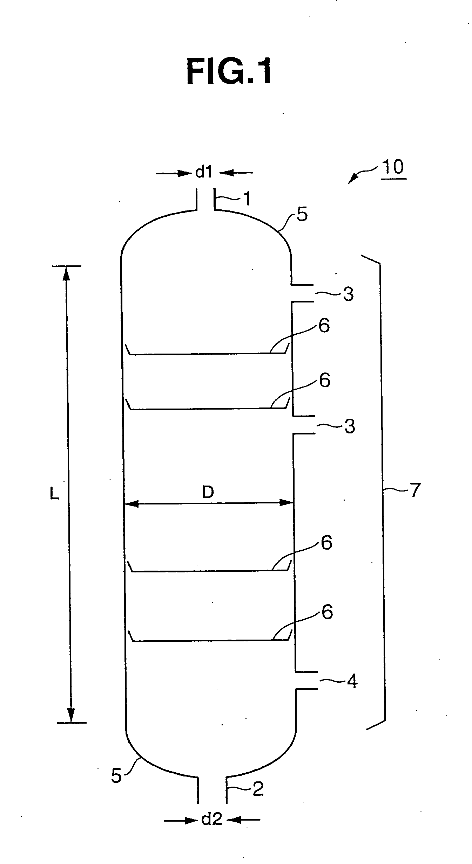

FIG. 1 is a schematic view of the continuous multi-stage distillation column for carrying out the present invention, the distillation column having an internal provided inside a trunk portion thereof.

Description of Reference Numerals:

1:    gas outlet
2:    liquid outlet
3:    inlet
4:    inlet
5:    end plate
6:    internal
7:    trunk portion
10:   continuous multi-stage distillation column

L:      length of trunk portion (cm)
D:      inside diameter of trunk portion (cm)
$d_1$:  inside diameter of gas outlet (cm)
$d_2$:  inside diameter of liquid outlet (cm)

Best Mode for Carrying out the Invention

In the following, the present invention is described in detail.
The dialkyl carbonate used in the present invention is a compound represented by the general formula (8);

$$R^1OCOOR^1 \qquad (8)$$

wherein $R^1$ represents an alkyl group having 1 to 10 carbon atoms, an alicyclic group having 3 to 10 carbon atoms, or an aralkyl group having 6 to 10 carbon atoms. Examples of $R^1$ include an alkyl group such as methyl, ethyl, propyl (isomers), allyl, butyl (isomers), butenyl (isomers), pentyl (isomers), hexyl (isomers), heptyl (isomers), octyl (isomers), nonyl (isomers), decyl (isomers) and cyclohexylmethyl; an alicyclic group such as cyclopropyl, cyclobutyl, cyclopentyl, cyclohexyl and cycloheptyl; and an aralkyl group such as benzyl, phenethyl (isomers), phenylpropyl (isomers), phenylbutyl (isomers) and methylbenzyl (isomers). The above-mentioned alkyl group, alicyclic group and aralkyl group may be substituted with other substituents such as a lower alkyl group, a lower alkoxy group, a cyano group or a halogen atom, and may also contain an unsaturated bond therein.
Examples of dialkyl carbonates having such $R^1$ include dimethyl carbonate, diethyl carbonate, dipropyl carbonate (isomers), diallyl carbonate, dibutenyl carbonate (isomers), dibutyl carbonate (isomers), dipentyl carbonate (isomers), dihexyl carbonate (isomers), diheptyl carbonate (isomers), dioctyl carbonate (isomers), dinonyl carbonate (isomers), didecyl carbonate (isomers), dicyclopentyl carbonate, dicyclohexyl carbonate, dicycloheptyl carbonate, dibenzyl carbonate, diphenethyl carbonate (isomers), di(phenylpropyl) carbonate (isomers), di(phenylbutyl) carbonate (isomers), di(chlorobenzyl) carbonate (isomers), di(methoxybenzyl) carbonate (isomers), di(methoxymethyl) carbonate, di(methoxyethyl) carbonate (isomers), di(chloroethyl) carbonate (isomers) and di(cyanoethyl) carbonate (isomers).
Of these dialkyl carbonates, ones preferably used in the present invention are dialkyl carbonates in which $R^1$ is an alkyl group having not more than four carbon atoms and not containing a halogen atom. A particularly preferable one is dimethyl carbonate. Moreover, of preferable dialkyl carbonates, particularly preferable ones are dialkyl carbonates produced in a state substantially not containing a halogen atom, for example, ones produced from an alkylene carbonate substantially not containing a halogen atom and an alcohol substantially not containing a halogen atom.
An aromatic monohydroxy compound used in the present invention is a compound represented by the following general formula (9). The type of the aromatic monohydroxy compound is not limited, as long as the hydroxyl group is directly bonded to the aromatic group;

$$Ar^1OH \qquad (9)$$

wherein $Ar^1$ represents an aromatic group having 5 to 30 carbon atoms. Examples of the aromatic monohydroxy compounds having such $Ar^1$ include phenol; various alkylphenols such as, cresol (isomers), xylenol (isomers), trimethylphenol (isomers), tetramethylphenol (isomers), ethylphenol (isomers), propylphenol (isomers), butylphenol (isomers), diethylphenol (isomers), methylethylphenol (isomers), methylpropylphenol (isomers), dipropylphenol (isomers), methylbutylphenol (isomers), pentylphenol (isomers), hexylphenol (isomers) and cyclohexylphenol (isomers); various alkoxyphenols such as methoxyphenol (isomers) and ethoxyphenol (isomers); arylalkylphenols such as phenylpropylphenol (isomers); naphthol (isomers) and various substituted naphthols; and heteroaromatic monohydroxy compounds such as hydroxypyridine (isomers), hydroxycoumarin (isomers) and hydroxyquinoline (isomers).
Of these aromatic monohydroxy compounds, ones preferably used in the present invention are aromatic monohydroxy compounds in which $Ar^1$ is an aromatic group having 6 to 10 carbon atoms. Phenol is particularly preferable. Moreover, of these aromatic monohydroxy compounds, ones substantially not containing a halogen atom are preferably used in the present invention.
The molar ratio of the dialkyl carbonate to the aromatic monohydroxy compound used as the starting material in the present invention must be in a range of from 0.4 to 4. Outside this range, the amount of unreacted starting materials remaining, based on a prescribed amount of the aromatic carbonate produced, will become high, which is not efficient for the production of the aromatic carbonate. Moreover, much energy will be required to recover this unreacted starting material. For such reasons, the above molar ratio is more preferably in a range of from 0.5 to 3, yet more preferably 0.8 to 2.6, most preferably 1.0 to 2.0.
In the present invention, not less than 1 ton per hour of the aromatic carbonate is produced continuously. The minimum amount of the aromatic monohydroxy compound fed into continuously for the above production is generally 13P ton/hr, preferably 10P ton/hr, more preferably 7P ton/hr, based on the amount of the aromatic carbonate (P ton/hr) to be produced. More preferably, this amount can be made to be less than 7P ton/hr.

The dialkyl carbonate and the aromatic monohydroxy compound as the starting material used in the present invention must contain specified amounts of the alcohol and the aromatic carbonate which are reaction products. Since the present reaction is an equilibrium reaction, it has been thought in the past that use of the starting material containing the alcohol and the aromatic carbonate which are the reaction products would be disadvantageous in terms of chemical equilibrium. However, it was discovered that in the case of using the continuous multi-stage distillation column according to the present invention, even if the starting material contains 0.01 to 1 % by weight of the alcohol and 0.01 to 5 % by weight of the aromatic carbonate, based on the total weight of the starting material, then surprisingly the alcohol and the aromatic carbonate have hardly any influence on the production of the aromatic carbonate. A more preferable alcohol content is 0.05 to 0.8 % by weight, with 0.1 to 0.5 % by weight being yet more preferable. Moreover, a more preferable aromatic carbonate content is 0.1 to 4 % by weight, with 0.5 to 3 % by weight being yet more preferable.

The reaction between the aromatic carbonate and the alcohol which is the reverse reaction to the present reaction has a very high equilibrium constant, and the reaction rate is high. With a batch reaction system in which the gas-liquid interfacial area is small, it can thus be easily understood that use of the starting material containing the alcohol and the aromatic carbonate which are the reaction products would be very disadvantageous in terms of chemical equilibrium for producing the aromatic carbonate for which the reverse reaction readily occurs. Moreover, with a small-scale reactive distillation system, the residence time of the reaction liquid is generally short, and hence it is clear that use of the starting material containing the alcohol and the aromatic carbonate which are the reaction products would be disadvantageous in terms of chemical equilibrium for producing the aromatic carbonate, since there would be little decrease in the concentrations of the alcohol and the aromatic carbonate in the reaction liquid.

However, it was discovered that if the continuous multi-stage distillation column according to the present invention is used, surprisingly this disadvantageous effect hardly occurs at all. The precise reason for this is unclear, but it is supposed that this is because the aromatic carbonate as the starting material introduced into the upper portion of the column reacts with the alcohol which is present at a high concentration at the stage in the upper portion of the column, and is thus rapidly converted into the dialkyl carbonate and the aromatic monohydroxy compound. It is also supposed that this is because in the continuous multi-stage distillation column according to the present invention, the residence time is sufficiently long for this reaction to take place in the upper portion of the column. Moreover, it is supposed that some or most of the alcohol in the starting material fed into the distillation column is used in this reaction, and the remainder moves into the vapor phase efficiently. This effect has been discovered for the first time through prolonged continuous stable operation using the industrial-scale continuous multi-stage distillation column according to the present invention.

When implementing the present reaction industrially, besides fresh dialkyl carbonate and aromatic monohydroxy compound newly introduced into the reaction system, it is preferable to be able to use as the starting material a material containing mainly the dialkyl carbonate and the aromatic monohydroxy compound that has been recovered in this process and/or another process. The present invention makes this possible, which is an excellent characteristic feature of the present invention.

The starting material in the present invention may contain compounds or reaction by-products, for example, an alkyl aryl ether or a high boiling point by-product, which are produced in this process and/or another process. It has been found that a starting material containing 0.5 to 15 % by weight of an alkyl aryl ether, based on the total weight of the starting material is preferable when implementing the present invention. A more preferable range for the alkyl aryl ether content in the starting material is 2 to 12 % by weight, with 4 to 10 % by weight being yet more preferable.

In the present invention, in the case, for example, of producing methyl phenyl carbonate and diphenyl carbonate using as the starting material a mixture of dimethyl carbonate as the dialkyl carbonate and phenol as the aromatic monohydroxy compound, it is preferable for this starting material to contain methanol, and methyl phenyl carbonate and diphenyl carbonate, which are the reaction products, in amounts as described above. Further, it is preferable for the starting material to contain anisole, which is a reaction by-product, in an amount as described above. Furthermore, the starting material may contain a high boiling point by-product such as the denaturated products of diphenyl carbonate or the like.

The aromatic carbonate produced in the present invention is an alkyl aryl carbonate, or a diaryl carbonate, and a mixture thereof, which are obtained through the transesterification reaction between the dialkyl carbonate and the aromatic monohydroxy compound. Included under this transesterification reaction are a reaction in which one or both of the alkoxy groups of the dialkyl carbonate is/are exchanged with the aryloxy group of the aromatic monohydroxy compound and an alcohol is eliminated, and a reaction in which two molecules of the alkyl aryl carbonate produced are converted into the diaryl carbonate and the dialkyl carbonate through a transesterification reaction therebetween, i.e. a disproportionation. In the present invention, although the alkyl aryl carbonate is mainly obtained, this can be converted into the diaryl carbonate by making the alkyl aryl carbonate further undergo a transesterification reaction with the aromatic monohydroxy compound, or by making the alkyl aryl carbonate further undergo a disproportionation. Since this diaryl carbonate does not contain a halogen atom at all, it is important as the raw material when industrially producing polycarbonate by means of the transesterification process.

As a catalyst used in the present invention, for example, a metal-containing compound selected from the following compounds can be used;

<lead compounds>:

lead oxides such as $PbO$, $PbO_2$ and $Pb_3O_4$, lead sulfides such as $PbS$ and $Pb_2S$; lead hydroxides such as $Pb(OH)_2$ and $Pb_2O_2(OH)_2$; plumbites such as $Na_2Pb0_2$, $K_2PbO_2$, $NaHPbO_2$ and $KHPbO_2$; plumbates such as $Na_2PbO_3$, $Na_2H_2PbO_4$, $K_2PbO_3$, $K_2[Pb(OH)_6]$, $K_4PbO_4$, $Ca_2PbO_4$ and $CaPbO_3$; lead carbonates and basic salts thereof such as $PbCO_3$ and $2PbCO_3 \cdot Pb(OH)_2$; lead salts of organic acids, and carbonates and basic salts thereof, such as $Pb(OCOCH_3)_2$, $Pb(OCOCH_3)_4$ and $Pb(OCOCH_3)_2 \cdot PbO \cdot 3H_2O$, organolead compounds such as $Bu_4Pb$, $Ph_4Pb$, $Bu_3PbCl$, $Ph_3PbBr$, $Ph_3Pb$ (or $Ph_6Pb_2$), $Bu_3PbOH$ and $Ph_3PbO$ (where Bu represents a butyl group, and Ph represents a phenyl group); alkoxylead compounds and aryloxylead compounds such as $Pb(OCH_3)_2$, $(CH_3O)Pb(OPh)$ and $Pb(OPh)_2$; lead alloys such as Pb-Na, Pb-Ca, Pb-Ba, Pb-Sn and Pb-Sb; lead minerals such as galena and zinc blende; and hydrates of such lead compounds;

<copper family metal compounds>:

salts and complexes of copper family metals such as $CuCl$, $CuCl_2$, $CuBr$, $CuBr_2$, $CuI$, $CuI_2$, $Cu(OAc)_2$, $Cu(acac)_2$, copper oleate, $Bu_2Cu$, $(CH_3O)_2Cu$, $AgNO_3$, $AgBr$, silver picrate, $AgC_6H_6ClO_4$, $[AuC\equiv C-C(CH_3)_3]_n$ and $[Cu(C_7H_8)Cl]_4$ (wherein acac represents an acetylacetone chelate ligand);

<alkali metal complexes>:

alkali metal complexes such as $Li(acac)$ and $LiN(C_4H_9)_2$;

<zinc complexes>:

zinc complexes such as $Zn(acac)_2$;

<cadmium complexes>:

cadmium complexes such as $Cd(acac)_2$;

<iron family metal compounds>:

complexes of iron family metals such as $Fe(C_{10}H_8)(CO)_5$, $Fe(CO)_5$, $Fe(C_4H_6)(CO)_3$, $Co(mesitylene)_2$, $(PEt_2Ph_2)$, $CoC_5F_5(CO)_7$, $Ni-\pi-C_5H_5NO$ and ferrocene;

<zirconium complexes>:

zirconium complexes such as $Zr(acac)_4$ and zirconocene;

<Lewis acid type compounds>:

Lewis acids and Lewis acid-forming transition metal compounds such as $AlX_3$, $TiX_3$, $TiX_4$, $VOX_3$, $VX_5$, $ZnX_2$, $FeX_3$ and $SnX_4$ (wherein X represents a halogen atom, an acetoxy group, an alkoxy group or an aryloxy group); and

<organotin compounds>:

organotin compounds such as $(CH_3)_3SnOCOCH_3$, $(C_2H_5)_3SnOCOC_6H_5$, $Bu_3SnOCOCH_3$, $Ph_3SnOCOCH_3$, $Bu_2Sn(OCOCH_3)_2$, $Bu_2Sn(OCOC_{11}H_{23})_2$, $Ph_3SnOCOCH_3$, $(C_2H_5)_3SnOPh$, $Bu_2Sn(OCH_3)_2$, $Bu_2Sn(OC_2Hs)_2$, $Bu_2Sn(OPh)_2$, $Ph_2Sn(OCH_3)_2$, $(C_2H_5)_3SnOH$, $Ph_3SnOH$, $Bu_2SnO$, $(C_8H_{17})_2SnO$, $Bu_2SnCl_2$ and $BuSnO(OH)$.

Each of these catalysts may be a solid catalyst fixed inside the multi-stage distillation column, or may be a soluble catalyst that dissolves in the reaction system.

Each of these catalyst components may of course have been reacted with an organic compound present in the reaction system such as an aliphatic alcohol, the aromatic monohydroxy compound, the alkyl aryl carbonate, the diaryl carbonate or the dialkyl carbonate, or may have been subjected to heating treatment with the starting material or the products prior to the reaction.

In the case of carrying out the present invention with a soluble catalyst which dissolves in the reaction system, the catalyst is preferably one having a high solubility in the reaction liquid under the reaction conditions. Examples of preferable catalysts in this sense include $PbO$, $Pb(OH)_2$ and $Pb(OPh)_2$; $TiCl_4$, $Ti(OMe)_4$, $(MeO)Ti(OPh)_3$, $(MeO)_2Ti$ $(OPh)_2$, $(MeO)_3Ti(OPh)$ and $Ti(OPh)_4$; $SnCl_4$, $Sn(OPh)_4$, $Bu_2SnO$ and $Bu_2Sn(OPh)_2$; $FeCl_3$, $Fe(OH)_3$ and $Fe(OPh)_3$; or such catalysts which have been treated with phenol, the reaction liquid or the like.

Fig. 1 shows a schematic view of the continuous multi-stage distillation column 10 for carrying out the present invention, the distillation column having an internal 6 provided inside a trunk portion 7 thereof. The continuous multi-stage distillation column 10 according to the present invention comprises a structure having a pair of end plates 5 above and below a cylindrical trunk portion 7 having a length L (cm) and an inside diameter D (cm) and having an internal 6 with a number of stages n thereinside, and further comprises a gas outlet 1 having an inside diameter $d_1$ (cm) at the top of the column or in an upper portion of the column near to the top, a liquid outlet 2 having an inside diameter $d_2$ (cm) at the bottom of the column or in a lower portion of the column near to the bottom, at least one inlet 3 in the upper portion and/or a central portion of the column below the gas outlet 1, and at least one inlet 4 in the lower portion of the column above the liquid outlet 2. Note that since Fig. 1 shows one embodiment of the continuous multi-stage distillation column according to the present invention, an arrangement of the internal is not limited to that of Fig. 1.

Moreover, the continuous multi-stage distillation column 10 according to the present invention must be made to satisfy various conditions so as to be able to carry out not only distillation but also reaction at the same time so as to be able to produce not less than 1 ton of an aromatic carbonate per hour stably for a prolonged period of time. That is, the continuous multi-stage distillation column according to the present invention meets not only conditions from the perspective of the distillation function, but also the combined conditions required so as to make the reaction proceed stably and with high selectivity.

More specifically, the followings are required for the continuous multi-stage distillation column according to the present invention:

(1) the length L (cm) must satisfy formula (1),

$$1500 \leqq L \leqq 8000 \qquad (1),$$

(2) the inside diameter D (cm) of the column must satisfy formula (2),

$$100 \leqq D \leqq 2000 \qquad (2),$$

(3) the ratio of the length L (cm) to the inside diameter D (cm) of the column must satisfy formula (3),

$$2 \leqq L/D \leqq 40 \qquad (3),$$

(4) the number of stages n must satisfy formula (4),

$$20 \leqq n \leqq 120 \qquad (4),$$

(5) the ratio of the inside diameter D (cm) of the column to the inside diameter $d_1$ (cm) of the gas outlet must satisfy formula (5),

$$5 \leqq D/d_1 \leqq 30 \qquad (5),$$

and

(6) the ratio of the inside diameter D (cm) of the column to the inside diameter $d_2$ (cm) of the liquid outlet must satisfy formula (6),

$$3 \leqq D / d_2 \leqq 20 \qquad (6).$$

It should be noted that the term "in an upper portion of the column near to the top" refers to the portion extending downwardly from the top of the column to the location measuring about 0.25 L, and the term "in a lower portion of the column near to the bottom" refers to the portion extending upwardly from the bottom of the column to the location measuring about 0.25L. Note that L is defined above.

It has been discovered that by using the continuous multi-stage distillation column that simultaneously satisfies formulae (1), (2), (3), (4), (5) and (6), the aromatic carbonate can be produced from the dialkyl carbonate and the aromatic monohydroxy compound on an industrial scale of not less than 1 ton per hour with high selectivity and high productivity stably for a prolonged period of time, for example, not less than 2000 hours, preferably not less than 3000 hours, more preferably not less than 5000 hours. The reason why it has become possible to produce the aromatic carbonate on the industrial scale with such excellent effects by implementing the process according to the present invention is not clear, but this is supposed to be due to a combined effect brought about when the conditions of the formulae (1) to (6) are combined. Preferable ranges for the respective factors are described below.

If L (cm) is less than 1500, then the reaction ratio decreases and it is not possible to attain the desired production amount. Moreover, to keep down the equipment cost while securing the reaction ratio enabling the desired production amount to be attained, L must be made to be not more than 8000. A more preferable range for L (cm) is $2000 \leqq L \leqq 6000$, with $2500 \leqq L \leqq 5000$ being yet more preferable.

If D (cm) is less than 100, then it is not possible to attain the desired production amount. Moreover, to keep down the equipment cost while attaining the desired production amount, D must be made to be not more than 2000. A more preferable range for D (cm) is $150 \leqq D \leqq 1000$, with $200 \leqq D \leqq 800$ being yet more preferable.

If L/D is less than 2 or greater than 40, then stable operation becomes difficult. In particular, if L / D is greater than 40, then the pressure difference between the top and bottom of the column becomes too great, and hence prolonged stable operation becomes difficult. Moreover, it is necessary to increase the temperature in the lower portion of the column, and hence side reactions become liable to occur, thereby bringing about a decrease in the selectivity. A more preferable range for L / D is $3 \leqq L / D \leqq 30$, with $5 \leqq L/ D \leqq 15$ being yet more preferable.

If n is less than 20, then the reaction ratio decreases and it is not possible to attain the desired production amount. Moreover, to keep down the equipment cost while securing the reaction ratio enabling the desired production amount to be attained, n must be made to be not more than 120. Furthermore, if n is greater than 120, then the pressure difference between the top and bottom of the column becomes too great, and hence prolonged stable operation becomes difficult. Moreover, it is be necessary to increase the temperature in the lower portion of the column, and hence side reactions become liable to occur, thereby bringing about a decrease in the selectivity. A more preferable range for n is $30 \leqq n \leqq 100$, with $40 \leqq n \leqq 90$ being yet more preferable.

If $D / d_1$ is less than 5, then the equipment cost becomes high. Moreover, since large amounts of gaseous components are readily released to the outside of the system, the stable operation becomes difficult. If $D / d_1$ is greater than 30, then the gaseous component withdrawal amount becomes relatively low. Moreover, the stable operation becomes difficult, and a decrease in the reaction ratio is brought about. A more preferable range for $D / d_1$ is $8 \leqq D / d_1 \leqq 25$, with $10 \leqq D / d_1 \leqq 20$ being yet more preferable.

If $D / d_2$ is less than 3, then the equipment cost becomes high. Moreover, the liquid withdrawal amount becomes relatively high, and hence stable operation becomes difficult. If $D / d_2$ is greater than 20, then the flow rate through the liquid outlet and piping become excessively fast, and erosion becomes liable to occur, thereby bringing about corrosion of the apparatus. A more preferable range for $D / d_2$ is $5 \leqq D/d_2 \leqq 18$, with $7 \leqq D/d2 \leqq 15$ being yet more preferable.

Furthermore, it has been found in the present invention that it is further preferable for $d_1$ and $d_2$ to satisfy the formula (7)

$$1 \leqq d_2/ d_1 \leqq 5 \qquad (7).$$

The term "prolonged stable operation" used in the present invention means that operation has been carried out continuously in a steady state for not less than 1000 hours, preferably not less than 3000 hours, more preferably not less than 5000 hours without clogging of piping, erosion and so on, and a prescribed amount of the aromatic carbonate has been produced while maintaining high selectivity.

A characteristic feature of the present invention is that at least one aromatic carbonate can be produced stably for a prolonged period of time with high selectivity and with a high productivity of not less than 1 ton per hour, preferably not less than 2 tons per hour, more preferably not less than 3 tons per hour. Moreover, other characteristic feature of the present invention is that in the case that L, D, L / D, n, D / $d_1$, and D / $d_2$ for the continuous multi-stage distillation column satisfy the following formulae; $2000 \leqq L \leqq 6000$, $150 \leqq D \leqq 1000$, $3 \leqq L/D \leqq 30$, $30 \leqq n \leqq 100$, $8 \leqq D/d_1 \leqq 25$, and $5 \leqq D/d_2 \leqq 18$, respectively, not less than 2 tons per hour, preferably not less than 2.5 tons per hour, more preferably not less than 3 tons per hour of the aromatic carbonate can be produced. Furthermore, another characteristic feature of the present invention is that in the case that L, D, L / D, n, D / $d_1$, and D / $d_2$ for the continuous multi-stage distillation column satisfy the following formulae; $2500 \leqq L \leqq 5000$, $200 \leqq D \leqq 800$, $5 \leqq L/D \leqq 15$, $40 \leqq n \leqq 90$, $10 \leqq D/d_1 \leqq 25$, and $7 \leqq D/d_2 \leqq 15$, respectively, not less than 3 tons per hour, preferably not less than 3.5 tons per hour, more preferably not less than 4 tons per hour of the aromatic carbonate can be produced.

"Selectivity for the aromatic carbonate" used in the present invention is based on the aromatic monohydroxy compound reacted. In the present invention, a high selectivity of not less than 95% can generally be attained, preferably not less than 97%, more preferably not less than 99%.

The continuous multi-stage distillation column according to the present invention is preferably a distillation column having a tray and/or a packing as the internal. The term "internal" used in the present invention means the part in the distillation column where gas and liquid are actually brought into contact one another. As the tray, for example, a bubble-cap tray, a sieve tray, a valve tray, a counterflow tray, a Superfrac tray, a Maxfrac tray or the like are preferable. As the packing, irregular packings such as a Raschig ring, a Lessing ring, a Pall ring, a Berl saddle, a Intalox saddle, a Dixon packing, a McMahon packing or Heli Pak, or regular packings such as Mellapak, Gempak, TECHNO-PAK, FLEXI-PAK, a Sulzer packing, a Goodroll packing or a Glitchgrid are preferable. The multi-stage distillation column having both a tray portion and a portion packed with packing can also be used.

The term "number of stages (n) of an internal" used in the present invention means that the total number of trays in the case of a tray, and the theoretical number of stages in the case of a packing.

Although the reaction between the dialkyl carbonate and the aromatic monohydroxy compound in the present invention has an extremely low equilibrium constant and the reaction rate is slow, it has been discovered that a plate-type distillation column having a tray as the internal is particularly preferable as the continuous multi-stage distillation column used in the reactive distillation. Furthermore, it has been discovered that a sieve tray having a sieve portion and a down corner portion is particularly preferable as the tray in terms of the relationship between performace thereof and the equipment cost. It has been also discovered that the sieve tray preferably has 100 to 1000 holes/m$^2$ in the sieve portion. A more preferable number of holes is 120 to 900 holes/m$^2$, yet more preferably 150 to 800 holes/m$^2$. Moreover, it was discovered that the cross-sectional area per hole of the sieve tray is preferably in a range of from 0.5 to 5 cm$^2$. A more preferable cross-sectional area per hole is 0.7 to 4 cm$^2$, yet more preferably 0.9 to 3 cm$^2$. Furthermore, it has been discovered that it is particularly preferable if the sieve tray has 100 to 1000 holes/m$^2$ in the sieve portion, and the cross-sectional area per hole is in a range of from 0.5 to 5 cm$^2$. It has been shown that by adding the above conditions to the continuous multi-stage distillation column, the object of the present invention can be attained more easily.

When carrying out the present invention, at least one aromatic carbonate can be produced continuously by continuously feeding a mixture of the dialkyl carbonate and the aromatic monohydroxy compound as the starting material into the continuous multi-stage distillation column in which a catalyst is present, carrying out reaction and distillation simultaneously in the column, continuously withdrawing a low boiling point reaction mixture containing a produced alcohol from the upper portion of the column in a gaseous form, and continuously withdrawing a high boiling point reaction mixture containing the at least one aromatic carbonate from the lower portion of the column in a liquid form. One of the characteristic features of the present invention is that the starting material contains the alcohol, the alkyl aryl carbonate, the diaryl carbonate and/or the alkyl aryl ether which are reaction products.

Moreover, in the present invention, when continuously feeding the mixture of the dialkyl carbonate and the aromatic monohydroxy compound is carried out, which is the starting material fed into the continuous multi-stage distillation column, this starting material may be fed thereinto in a liquid form and/or a gaseous form from inlet(s) provided in one or a plurality of positions in the upper portion or the central portion of the column below the gas outlet in the upper portion of the distillation column. It is also preferable to feed the starting material containing a large proportion of the aromatic monohydroxy compound in a liquid form from the inlet provided in the upper portion of the distillation column and to feed the starting material containing a large proportion of the dialkyl carbonate in a gaseous form from the inlet provided in the lower portion of the column above the liquid outlet in the lower portion of the distillation column.

In the present invention, the method of making the catalyst be present in the continuous multi-stage distillation column may be any method, but in the case that the catalyst is a solid that is insoluble in the reaction liquid, there is, for example, a method in which the catalyst is fixed inside the column by, for example, being installed on a plate inside the continuous multi-stage distillation column or being installed in the form of packing. In the case of a catalyst that dissolves in the starting material or the reaction liquid, it is preferable to feed the catalyst into the distillation column from the position above the middle portion of the distillation column. In this case, the catalyst liquid dissolved in the starting material or reaction liquid may be introduced into the column together with the starting material, or may be introduced into the column from a different inlet from the starting material. The amount of the catalyst used in the present invention varies depending on the type thereof, the types and proportions of the starting material compounds, and reaction conditions such as the reaction temperature and the reaction pressure. Generally the amount of the catalyst is in a range of from 0.0001 to 30 % by weight, preferably 0.005 to 10 % by weight, more preferably 0.001 to 1 % by weight, based on the total weight of the starting material.

The reaction time for the transesterification carried out in the present invention is considered to equate to the average residence time of the reaction liquid in the continuous multi-stage distillation column. The reaction time varies depending on the type of the internal inside the distillation column and the number of stages, the amounts fed into the column of the starting material compounds, the type and amount of the catalyst, the reaction conditions, and so on. Generally, the reaction time is in a range of from 0.1 to 10 hours, preferably 0.3 to 5 hours, more preferably 0.5 to 3 hours.

The reaction temperature varies depending on the type of the starting material compounds used, and the type and amount of the catalyst. Generally the reaction temperature is in a range of from 100 to 350°C. It is preferable to increase the reaction temperature so as to increase the reaction rate. If the reaction temperature is too high, then side reactions become liable to occur, for example production of by-products such as an alkyl aryl ether increases, which is undesirable. For this reason, the reaction temperature is preferably in a range of from 130 to 280°C, more preferably 150 to 260°C, yet more preferably 180 to 250°C. Moreover, the reaction pressure varies depending on the type of the starting material compounds used and the composition of the starting material, the reaction temperature and so on. The reaction pressure may be any of a reduced pressure, normal pressure, or an applied pressure. Generally the reaction pressure is in a range of from 0.1 to $2\times10^7$ Pa, preferably $10^5$ to $10^7$ Pa, more preferably $2\times10^5$ to $5\times10^6$ Pa.

The material constituting the continuous multi-stage distillation column in the present invention is formed is generally a metallic material such as carbon steel or stainless steel. In terms of the quality of the aromatic carbonate produced, stainless steel is preferable.

Hereinbelow, the present invention will be described in more detail with reference to the following Examples, but the present invention is not limited to the following Examples.

Examples

A halogen content was measured by means of ion chromatography method.

<Continuous Multi-Stage Distillation Column>

A continuous multi-stage distillation column as shown in FIG. 1 having L = 3300 cm, D = 500 cm, L / D = 6.6, n = 80, D / $d_1$ = 17, and D / $d_2$ = 9 was used in the following Examples and Reference Example. In these Examples, as the internal, use was made of the sieve tray having the cross-sectional area per hole being approximately 1.5 $cm^2$ and the number of holes being approximately 250/$m^2$.

Example 1:

<Reactive Distillation>

A starting material 1 containing phenol and dimethyl carbonate in a weight ratio of phenol/dimethyl carbonate = 1.9 was introduced continuously in a liquid form at a flow rate of 50 ton/hr from an upper inlet of the distillation column. The starting material 1 contained 0.3 % by weight of methyl alcohol, 0.9 % by weight of methyl phenyl carbonate, 0.4 % by weight of diphenyl carbonate, and 7.3 % by weight of anisole. On the other hand, a starting material 2 containing dimethyl carbonate and phenol in a weight ratio of dimethyl carbonate / phenol = 3.6 was introduced continuously in a gaseous form at a flow rate of 50 ton/hr from a lower inlet of the distillation column. The starting material 2 contained 0.2 % by weight of methyl alcohol, 1.1 % by weight of methyl phenyl carbonate, and 5.1 % by weight of anisole. The molar ratio for the starting materials introduced into the distillation column was dimethyl carbonate / phenol = 1.35. The overall starting materials introduced into the distillation column contained 0.25 % by weight of methyl alcohol, 1.0 % by weight of methyl phenyl carbonate, 0.2 % by weight of diphenyl carbonate, and 6.2 % by weight of anisole. The starting materials substantially did not contain halogens (outside the detection limit for the ion chromatography, i.e. 1 ppb or less).

Pb(OPh)$_2$ as a catalyst was introduced from the upper portion of the column such that a concentration thereof in the reaction liquid would be approximately 100 ppm. Reactive distillation was carried out continuously under the conditions of a temperature at the bottom of the column being 225°C and a pressure at the top of the column being $7\times10^5$ Pa. It was possible to attain stable steady state operation after 24 hours. The liquid continuously withdrawn from the bottom of the column contained 18.2 % by weight of methyl phenyl carbonate and 0.8 % by weight of diphenyl carbonate. It was found that the amount of methyl phenyl carbonate produced per hour was 9.1 tons, and the amount of diphenyl carbonate produced per hour was 0.4 tons. The total selectivity for the methyl phenyl carbonate and diphenyl carbonate, based on the phenol reacted, was 99%. Note that in the present invention, although methyl phenyl carbonate and diphenyl carbonate were contained in the starting materials, since it can be supposed that these underwent reaction in the distillation column to ultimately give the above composition under the above conditions, a content of aromatic carbonate in the withdrawn liquid corresponds to the amount produced. (For example, the starting material 1 was introduced from the upper portion of the column, but the concentration of methyl alcohol would be high in the upper stage of the column, and hence it can be supposed that methyl phenyl carbonate and diphenyl carbonate were converted rapidly into dimethyl carbonate and phenol.)

Prolonged continuous operation was carried out under these conditions. The amounts produced per hour at 500 hours, 2000 hours, 4000 hours, 5000 hours, and 6000 hours after attaining stable steady state were 9.1 tons, 9.1 tons, 9.1 tons, 9.1 ton, and 9.1 tons, respectively for the methyl phenyl carbonate, and 0.4 tons, 0.4 tons, 0.4 tons, 0.4 tons, and 0.4 tons, respectively for the diphenyl carbonate. The total selectivities for the methyl phenyl carbonate and diphenyl carbonate were 99%, 99%, 99%, 99%, and 99%, respectively, and hence the operation was very stable. Moreover, the aromatic carbonates produced substantially did not contain halogens (1 ppb or less).

These results are the same as those obtained for Reference Example 1 which was carried out prior to Example 1, showing that the methyl alcohol, methyl phenyl carbonate, diphenyl carbonate and anisole in the starting materials had no influence on the production of the aromatic carbonate.

Reference Example 1:

This Reference Example was carried out prior to Example 1. Reactive distillation was carried out continuously under the same conditions as in Example 1 using the same continuous multi-stage distillation column as in Example 1, except that use was made of fresh starting materials not containing methyl alcohol, methyl phenyl carbonate, diphenyl carbonate and anisole. It was possible to attain stable steady state operation after 24 hours. The liquid continuously withdrawn from the bottom of the column contained 18.2 % by weight of methyl phenyl carbonate and 0.8 % by weight of diphenyl carbonate. It was found that the amount of methyl phenyl carbonate produced per hour was 9.1 tons, and the amount of diphenyl carbonate produced per hour was 0.4 tons. The total selectivity for the methyl phenyl carbonate and diphenyl carbonate, based on the phenol reacted, was 99%. Stable operation was also attained in exactly the same state after 50 hours, 100 hours, and 200 hours.

Example 2:

Reactive distillation was carried out under the following conditions using the same continuous multi-stage distillation column as in Example 1.

A starting material 1 containing phenol and dimethyl carbonate in a weight ratio of phenol/dimethyl/ carbonate =1.1 was introduced continuously in a liquid form at a flow rate of 40 ton/hr from the upper inlet of the distillation column. The starting material 1 contained 0.3 % by weight of methyl alcohol; 1.0 % by weight of methyl phenyl carbonate, and 5.6 % by weight of anisole. On the other hand, a starting material 2 containing dimethyl carbonate and phenol in a weight ratio of dimethyl carbonate / phenol = 3.9 was introduced continuously in a gaseous form at a flow rate of 43 ton/hr from the lower inlet of the distillation column. The starting material 2 contained 0.1 % by weight of methyl alcohol, 0.2 % by weight of methyl phenyl carbonate, and 4.4 % by weight of anisole. The molar ratio for the starting materials introduced into the distillation column was dimethyl carbonate / phenol = 1.87. The overall starting materials introduced into the distillation column contained 0.2 % by weight of methyl alcohol, 0.59 % by weight of methyl phenyl carbonate, and 5.0 % by weight of anisole. The starting materials substantially did not contain halogens (outside the detection limit for the ion chromatography, i.e. 1 ppb or less).

Pb(OPh)$_2$ as the catalyst was introduced from the upper portion of the column such that a concentration thereof in the reaction liquid would be approximately 250 ppm. Reactive distillation was carried out continuously under conditions of a temperature at the bottom of the column being 235°C and a pressure at the top of the column being $9 \times 10^5$ Pa. It was possible to attain stable steady state operation after 24 hours. The liquid continuously withdrawn from the bottom of the column contained 20.7 % by weight of methyl phenyl carbonate and 1.0 % by weight of diphenyl carbonate. It was found that the amount of methyl phenyl carbonate produced per hour was 8.3 tons, and the amount of diphenyl carbonate produced per hour was 0.4 tons. The total selectivity for the methyl phenyl carbonate and diphenyl carbonate, based on the phenol reacted, was 98%.

Prolonged continuous operation was carried out under these conditions. The amounts produced per hour at 500 hours, 1000 hours, and 2000 hours after attaining stable steady state were 8.3 tons, 8.3 tons, and 8.3 tons, respectively for the methyl phenyl carbonate, and 0.4 tons, 0.4 tons, and 0.4 tons, respectively for the diphenyl carbonate. The total selectivites for the methyl phenyl carbonate and diphenyl carbonate were 98%, 98%, and 98%, respectively, and hence the operation was very stable. Moreover, the aromatic carbonates produced substantially did not contain halogens (1 ppb or less).

Example 3:

Reactive distillation was carried out under the following conditions using the same continuous multi-stage distillation column as in Example 1.

A starting material 1 containing phenol and dimethyl carbonate in a weight ratio of phenol / dimethyl carbonate = 1.7 was introduced continuously in a liquid form at a flow rate of 86 ton/hr from the upper inlet of the distillation column. The starting material 1 contained 0.3 % by weight of methyl alcohol, 0.9 % by weight of methyl phenyl carbonate, 0.4 % by weight of diphenyl carbonate, and 7.3 % by weight of anisole. On the other hand, a starting material 2 containing dimethyl carbonate and phenol in a weight ratio of dimethyl carbonate / phenol = 3.5 was introduced continuously in a gaseous form at a flow rate of 90 ton/hr from the lower inlet of the distillation column. The starting material 2 contained 0.2 % by weight of methyl alcohol, 1.1 % by weight of methyl phenyl carbonate, and 5.1 % by weight of anisole. The molar ratio for the starting materials introduced into the distillation column was dimethyl carbonate / phenol = 1.44. The overall starting materials introduced into the distillation column contained 0.25 % by weight of methyl alcohol, 1.1 % by weight of methyl phenyl carbonate, 0.195 % by weight of diphenyl carbonate, and 6.17 % by weight of anisole. The starting materials substantially did not contain halogens (outside the detection limit for the ion chromatography, i.e. 1 ppb or less).

$Pb(OPh)_2$ as the catalyst was introduced from the upper portion of the column such that a concentration thereof in the reaction liquid would be approximately 150 ppm. Reactive distillation was carried out continuously under conditions of a temperature at the bottom of the column being 220°C and a pressure at the top of the column being $8 \times 10^5$ Pa. It was possible to attain stable steady state operation after 24 hours. The liquid continuously withdrawn from the bottom of the column contained 15.8 % by weight of methyl phenyl carbonate and 0.5 % by weight of diphenyl carbonate. It was found that the amount of methyl phenyl carbonate produced per hour was 12.8 tons, and the amount of diphenyl carbonate produced per hour was 0.4 tons. The total selectivity for the methyl phenyl carbonate and diphenyl carbonate, based on the phenol reacted, was 99%.

Prolonged continuous operation was carried out under these conditions. The amounts produced per hour at 500 hours, 1000 hours, and 2000 hours after attaining stable steady state were 12.8 tons, 12.8 tons, and 12.8 tons, respectively for the methyl phenyl carbonate, and 0.4 tons, 0.4 tons, and 0.4 tons, respectively for the diphenyl carbonate. The total selectivities for the methyl phenyl carbonate and diphenyl carbonate were 99%, 99%, and 99%, respectively, and hence the operation was very stable. Moreover, the aromatic carbonates produced substantially did not contain halogens (1 ppb or less).

Industrial Applicability

According to the present invention, even if there is used the starting material comprising the dialkyl carbonate and the aromatic monohydroxy compound containing an alcohol and an aromatic carbonate, which has been considered to be disadvantageous for the production of the aromatic carbonates, contents of the alcohol and the aromatic carbonate being in a specified range, and a specified continuous multi-stage distillation column can be used to produce the aromatic carbonate on an industrial scale of not less than 1 ton per hour, preferably not less than 2 tons per hour, more preferably not less than 3 tons per hour, with high selectivity of not less than 95%, preferably not less than 97%, more preferably not less than 99%, stably for a prolonged period of time of not less than 2000 hours, preferably not less than 3000 hours, more preferably not less than 5000 hours.

**Claims**

1. A process for the production of an aromatic carbonate from a dialkyl carbonate and an aromatic monohydroxy compound as a starting material, which comprises the steps of:

   (i) continuously feeding said starting material into a continuous multi-stage distillation column in which a catalyst is present;
   (ii) carrying out reaction in the column to produce an alcohol and at least one aromatic carbonate; and
   (iii) continuously withdrawing a low boiling point reaction mixture containing said produced alcohol from an upper portion of the column in a gaseous form and continuously withdrawing a high boiling point reaction mixture containing said at least one aromatic carbonate from a lower portion of the column in a liquid form, wherein

      (a) said starting material

(1) has a molar ratio of the dialkyl carbonate to the aromatic monohydroxy compound in a range of from 0.4 to 4, and
(2) comprises 0.01 to 1 % by weight of the alcohol, and 0.01 to 5 % by weight of the aromatic carbonate, based on the total weight of said starting material;

(b) said continuous multi-stage distillation column comprises a structure having a pair of end plates above and below a cylindrical trunk portion having a length L (cm) and an inside diameter D (cm) and having an internal with a number of stages n thereinside, and comprises a gas outlet having an inside diameter $d_1$ (cm) at the top of the column or in an upper portion of the column near thereto, a liquid outlet having an inside diameter $d_2$ (cm) at the bottom of the column or in a lower portion of the column near thereto, at least one inlet provided in the upper portion and/or a central portion of the column below the gas outlet, and at least one inlet provided in the lower portion of the column above the liquid outlet, wherein

(1) said length L (cm) satisfies following formula (1),

$$1500 \leqq L \leqq 8000 \qquad (1),$$

(2) said inside diameter D (cm) of the column satisfies the following formula (2),

$$100 \leqq D \leqq 2000 \qquad (2),$$

(3) a ratio of said length L (cm) to said inside diameter D (cm) of the column satisfies the following formula (3),

$$2 \leqq L/D \leqq 40 \qquad (3),$$

(4) said number of stages n satisfies the following formula (4),

$$20 \leqq n \leqq 120 \qquad (4),$$

(5) a ratio of said inside diameter D (cm) of the column to said inside diameter $d_1$ (cm) of the gas outlet satisfies the following formula (5),

$$5 \leqq D / d_1 \leqq 30 \qquad (5),$$

and
(6) a ratio of said inside diameter D (cm) of the column to said inside diameter $d_2$ (cm) of the liquid outlet satisfies the following formula (6),

$$3 \leqq D / d_2 \leqq 20 \qquad (6).$$

2. The process according to claim 1, wherein distillation is carried out simultaneously in said step (ii).

3. The process according to claim 1 or 2, wherein said at least one aromatic carbonate is continuously produced and an amount of the aromatic 5 carbonate produced is not less than 1 ton per hour.

**4.** In a process for the industrial production of an aromatic carbonate in which at least one aromatic carbonate is produced continuously by continuously feeding a dialkyl carbonate and an aromatic monohydroxy compound as a starting material into a continuous multi-stage distillation column in which a catalyst is present, carrying out reaction and distillation simultaneously in the column, continuously withdrawing a low boiling point reaction mixture containing a produced alcohol from an upper portion of the column in a gaseous form, and continuously withdrawing a high boiling point reaction mixture containing said at least one aromatic carbonate from a lower portion of the column in a liquid form, the improvement in which

(a) said starting material fed continuously into said continuous multi-stage distillation column

(1) has a molar ratio of the dialkyl carbonate to the aromatic monohydroxy compound in a range of from 0.4 to 4, and
(2) comprises 0.01 to 1 % by weight of the alcohol, and 0.01 to 5 % by weight of the aromatic carbonate, based on the total weight of said starting material;

(b) said continuous multi-stage distillation column comprises a structure having a pair of end plates above and below a cylindrical trunk portion having a length L (cm) and an inside diameter D (cm) and having an internal with a number of stages n thereinside, and comprises a gas outlet having an inside diameter $d_1$ (cm) at the top of the column or in an upper portion of the column near thereto, a liquid outlet having an inside diameter $d_2$ (cm) at the bottom of the column or in a lower portion of the column near thereto, at least one inlet provided in the upper portion and/or a central portion of the column below the gas outlet, and at least one inlet provided in the lower portion of the column above the liquid outlet, wherein

(1) said length L (cm) satisfies following formula (1),

$$1500 \leqq L \leqq 8000 \qquad (1),$$

(2) said inside diameter D (cm) of the column satisfies the following formula (2),

$$100 \leqq D \leqq 2000 \qquad (2),$$

(3) a ratio of said length L (cm) to said inside diameter D (cm) of the column satisfies the following formula (3),

$$2 \leqq L / D \leqq 40 \qquad (3),$$

(4) said number of stages n satisfies the following formula (4),

$$20 \leqq n \leqq 120 \qquad (4),$$

(5) a ratio of said inside diameter D (cm) of the column to said inside diameter $d_1$ (cm) of the gas outlet satisfies the following formula (5),

$$5 \leqq D / d_1 \leqq 30 \qquad (5),$$

and
(6) a ratio of said inside diameter D (cm) of the column to said inside diameter $d_2$ (cm) of the liquid outlet satisfies the following formula (6),

$$3 \leqq D / d_2 \leqq 20 \qquad (6).$$

5. The process according to claim 4, wherein an amount of the aromatic carbonate produced is not less than 1 ton per hour.

6. The process according to any one of claims 1 to 5, wherein said starting material further comprises 0.5 to 15 % by weight of an alkyl aryl ether, based on the total weight of said starting material.

7. The process according to any one of claims 1 to 6, wherein $d_1$ and $d_2$ satisfy the following formula (7):

$$1 \leqq d_2 / d_1 \leqq 5 \qquad (7).$$

8. The process according to any one of claims 1 to 7, wherein L, D, L / D, n, D / $d_1$, and D / $d_2$ for said continuous multi-stage distillation column satisfy the following formulae; $2000 \leqq L \leqq 6000$, $150 \leqq D \leqq 1000$, $3 \leqq L/D \leqq 30$, $30 \leqq n \leqq 100$, $8 \leqq D/d_1 \leqq 25$, and $5 \leqq D/d_2 \leqq 18$, respectively.

9. The process according to any one of claims 1 to 8, wherein L, D, L / D, n, D / $d_1$, and D / $d_2$ for said continuous multi-stage distillation column satisfy the following formulae; $2500 \leqq L \leqq 5000$, $200 \leqq D \leqq 800$, $5 \leqq L/D \leqq 15$, $40 \leqq n \leqq 90$, $1 0 \leqq D/d_1 \leqq 25$, and $7 \leqq D/d_2 \leqq 15$, respectively.

10. The process according to any one of claims 1 to 9, wherein said continuous multi-stage distillation column is a distillation column having a tray and/or a packing as the internal.

11. The process according to claim 10, wherein said continuous multi-stage distillation column is a plate-type distillation column having a tray as the internal.

12. The process according to claim 10 or 11, wherein said tray is a sieve tray having a sieve portion and a down corner portion.

13. The process according to claim 12, wherein said sieve tray has 100 to 1000 holes/$m^2$ in the sieve portion.

14. The process according to claim 12 or 13, wherein the cross-sectional area per hole of said sieve tray is in a range of from 0.5 to 5 $cm^2$.

15. An aromatic carbonate comprising a halogen content of not more than 0.1 ppm, produced by the process according to any one of claims 1 to 14.

16. A continuous multi-stage distillation column for carrying out reaction and distillation, comprising:

a cylindrical trunk portion having a length L (cm) and an inside diameter D (cm);
a pair of end plates provided above and below the trunk portion;
an internal with a number of stages n inside the trunk portion;
a gas outlet which has an inside diameter $d_1$ (cm) and which being provided on the end plate at the top of the column or in an upper portion of the column near thereto;
a liquid outlet which has an inside diameter $d_2$ (cm) and which being provided on the end plate at the bottom of the column or in a lower portion of the column near thereto;
at least one inlet provided in the upper portion and/or a central portion of the column below the gas outlet; and
at least one inlet provided in the lower portion of the column above the liquid outlet,

wherein

(1) said length L (cm) satisfies following formula (1),

$$1500 \leqq L \leqq 8000 \qquad (1),$$

(2) said inside diameter D (cm) of the column satisfies the following formula (2),

$$100 \leqq D \leqq 2000 \qquad (2),$$

(3) a ratio of said length L (cm) to said inside diameter D (cm) of the column satisfies the following formula (3),

$$2 \leqq L / D \leqq 40 \qquad (3),$$

(4) said number of stages n satisfies the following formula (4),

$$20 \leqq n \leqq 120 \qquad (4),$$

(5) a ratio of said inside diameter D (cm) of the column to said inside diameter $d_1$ (cm) of the gas outlet satisfies the following formula (5),

$$5 \leqq D/d_1 \leqq 30 \qquad (5),$$

and
(6) a ratio of said inside diameter D (cm) of the column to said inside diameter $d_2$ (cm) of the liquid outlet satisfies the following formula (6),

$$3 \leqq D/d_2 \leqq 20 \qquad (6).$$

**17.** The column according to claim 16, wherein $d_1$ and $d_2$ satisfy the following formula (7):

$$1 \leqq d_2 / d_1 \leqq 5 \qquad (7).$$

**18.** The column according to claim 16 or 17, wherein L, D, L / D, n, $D/d_1$, and D / $d_2$ for said continuous multi-stage distillation column satisfy the following formulae; $2000 \leqq L \leqq 6000$, $150 \leqq D \leqq 1000$, $3 \leqq L/D \leqq 30$, $30 \leqq n \leqq 100$, $8 \leqq D/d_1 \leqq 25$, and $5 \leqq D/d_2 \leqq 18$, respectively.

**19.** The column according to any one of claims 16 to 18, wherein L, D, L / D, n, $D/d_1$, and $D/d_2$ for said continuous multi-stage distillation column satisfy the following formulae; $2500 \leqq L \leqq 5000$, $200 \leqq D \leqq 800$, $5 \leqq L/D \leqq 15$, $40 \leqq n \leqq 90$, $10 \leqq D/d_1 \leqq 25$, and $7 \leqq D/d_2 \leqq 15$, respectively.

**20.** The column according to any one of claims 16 to 19, wherein said continuous multi-stage distillation column is a distillation column having a tray and/or a packing as the internal.

**21.** The column according to claim 20, wherein said continuous multi-stage distillation column is a plate-type distillation column having a tray as the internal.

**22.** The column according to claim 20 or 21, wherein said tray is a sieve tray having a sieve portion and a down corner portion.

23. The column according to claim 22, wherein said sieve tray has 100 to 1000 holes/m$^2$ in the sieve portion.

24. The column according to claim 22 or 23, wherein the cross-sectional area per hole of said sieve tray is in a range of from 0.5 to 5 cm$^2$.

# FIG.1

<div align="center">

**INTERNATIONAL SEARCH REPORT**

</div>

| International application No. |
|---|
| PCT/JP2005/011280 |

**A. CLASSIFICATION OF SUBJECT MATTER**
Int.Cl$^7$  C07C68/06, B01D3/14, C07C69/96, C07B61/00

According to International Patent Classification (IPC) or to both national classification and IPC

**B.  FIELDS SEARCHED**

Minimum documentation searched (classification system followed by classification symbols)
Int.Cl$^7$  C07C68/06, B01D3/14, C07C69/96, C07B61/00

Documentation searched other than minimum documentation to the extent that such documents are included in the fields searched
Jitsuyo Shinan Koho          1922-1996   Jitsuyo Shinan Toroku Koho   1996-2005
Kokai Jitsuyo Shinan Koho    1971-2005   Toroku Jitsuyo Shinan Koho   1994-2005

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| A | JP 2004-131421 A  (Mitsubishi Chemical Corp.), 30 April, 2004 (30.04.04), Claims (Family: none) | 1-24 |
| A | JP 2003-300936 A  (Mitsui Chemicals, Inc.), 21 October, 2003 (21.10.03), Claims (Family: none) | 1-24 |
| A | WO 2002/40439 A2  (GENERAL ELECTRIC CO.), 23 May, 2002 (23.05.02), Claims & US 2002-107355 A1      & US 2002-156312 A1 & EP 1337503 A2           & JP 2004-526672 A | 1-24 |

☐ Further documents are listed in the continuation of Box C.     ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 July, 2005 (08.07.05) | 02 August, 2005 (02.08.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 51105032 A
- JP 56123948 A
- JP 56123949 A
- DE 2528412
- GB 1499530 A
- US 4182726 A
- JP 51075044 A
- DE 2552907
- US 4045464 A
- JP 54048733 A
- DE 2736062
- JP 54063023 A
- JP 60169444 A
- US 4554110 A
- JP 60169445 A
- US 4552704 A
- JP 62277345 A
- JP 1265063 A
- JP 57176932 A
- JP 1093560 A
- JP 57183745 A
- JP 58185536 A
- US 4410464 A
- JP 1265062 A
- JP 60173016 A
- US 4609501 A
- JP 1265064 A
- JP 61172852 A
- JP 54048732 A
- DE 736063
- US 4252737 A
- US 410464 A
- JP 56025138 A
- JP 61291545 A
- JP 3291257 A
- JP 4009358 A
- JP 4211038 A
- JP 4224547 A
- JP 4230242 A
- JP 4235951 A
- WO 0018720 A
- US 5362901 A
- IT 01255746
- JP 6009506 A
- EP 0560159 A
- US 5282965 A
- JP 6041022 A
- EP 0572870 A
- JP 6157424 A
- EP 0582931 A
- US 5334742 A
- JP 6184058 A
- EP 0582930 A
- US 5344954 A
- JP 7304713 A
- JP 9040616 A
- JP 9059225 A
- JP 9110805 A
- JP 9165357 A
- JP 9173819 A
- JP 9176094 A
- JP 2000191596 A
- JP 2000191597 A
- JP 9194436 A
- EP 0785184 A
- US 5705673 A
- US 6093842 A
- JP 2001064234 A
- JP 2001064235 A
- WO 0240439 A
- US 6596894 B
- US 6596895 B
- US 6600061 B
- WO 9711049 A
- EP 0855384 A
- US 5872275 A
- JP 9255772 A
- EP 0892001 A
- US 5747609 A